(19)

Europäisches
Patentamt
European
Patent Office
Office européen
des brevets

(11) **EP 3 220 146 A1**

(12) **EUROPEAN PATENT APPLICATION**

(43) Date of publication:
**20.09.2017  Bulletin 2017/38**

(51) Int Cl.:
*G01N 33/68* (2006.01)          *G01N 33/74* (2006.01)

(21) Application number: **16161039.9**

(22) Date of filing: **18.03.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Stichting Wageningen Research
6708 PB  Wageningen (NL)**

(72) Inventors:
 • **TE PAS, Marinus Frederik Willem
  3882 XG Putten (NL)**
 • **WOELDERS, Henri
  8212 EA Lelystad (NL)**
 • **BOUWMAN, Aniek Cornelia
  6708 NC Wageningen (NL)**
 • **HULSEGGE, Berendina
  3738 CX Maartensdijk (NL)**

(74) Representative: **Wensvoort, Gert
  Hoge Linthorst 1
  7958 NZ Koekange (NL)**

Remarks:
 Claims 16-29 are deemed to be abandoned due to
 non-payment of the claims fee (Rule 45(3) EPC).

(54)  **DETECTION OF PROPER INSEMINATION TIME BY MULTIPLE PREGNANCY MARKER PATTERN (MPMP) RECOGNITION**

(57)    The invention relates to methods for pregnancy and non-pregnancy testing and also relates to methods allowing determination of proper insemination time to establish pregnancy. The invention provides method to determine whether an inseminated female mammal is pregnant prior to a point in time at which said mammal would be expected to ovulate if non-pregnant, said method comprising taking a biological sample from said mammal prior to said point in time and testing said sample for the presence of a multiple pregnancy marker pattern (MPMP). It is preferred that said sample is tested for the relative expression of progesterone and of at least one marker selected from a group of marker proteins that each contribute to a significantly differential marker expression pattern related to the status pregnancy and is tested to determine whether said mammal is non-pregnant and may be re-inseminated.

**EP 3 220 146 A1**

**Description**

The invention relates to methods for pregnancy and non-pregnancy testing and also relates to methods allowing determination of proper insemination time to establish pregnancy.

Background

Profit for dairy farms depends upon successful reproduction. Milk is only produced when a cow delivers a calf, hence getting cows pregnant is essential. In theory, the process is simple - watch the cows and inseminate at standing heat - but in these days of large herds, high milk production, artificial inseminations and busy employees, the reality is that getting cows pregnant can be quite a challenge. Thus numerous approaches have been developed to increase the likelihood of and opportunities for successful reproduction of cows in heat. Estrus in cattle is commonly referred to as heat. Considering that ovulation in cows occurs at an interval of commonly 21 days, estrus or heat generally occurs from every 18 to 24 days in sexually mature, open (non-pregnant) female cattle. During heat, cows are receptive to mounting activity by bulls or other cows, generally displaying standing heat that predicts optimal estrus with highest readiness to be inseminated and highest chances on successful conception, at around day 21. Since cattle should be inseminated so that viable sperm are at the fertilization site as the unfertilized egg arrives, it is important to estimate the time of ovulation for each cow that is to be inseminated. Ovulation occurs generally 25 to 32 hours after the onset of standing heat. Standing behaviour is a reliable indicator producers have to determine time of ovulation.

Sperm have to be in the female reproductive tract for approximately six hours before they are capable of fertilizing the egg. This process is termed capacitation. Although live sperm have been found in the female tract up to 48 hours after insemination, sperm viability usually is estimated to be 18 to 24 hours.

The egg travels very rapidly from the ovulation site to the fertilization site in the oviduct. The fertile life of the egg is shorter than that of the sperm. Ovulated eggs remain fertilizable longer (10-20 hours) than they remain capable of being fertilized and developing into normal embryos (8-10 hours). Thus the likelihood of embryonic death increases as the time beyond this interval increases. Thus viable sperm should be at the site of fertilization awaiting the arrival of the freshly ovulated egg. Inseminating either too early or too late allows an aged sperm or an aged egg to interact at the site of fertilization and will result in poor conception.

Heat detection is critical to heat synchronization and inseminating programs, particularly to determine proper time slots for artificial insemination (AI) or embryo transfer (ET).

Effective heat detection is often the most limiting factor for reproduction. Visual observation is the commonly used method of heat detection. It involves a trained observer's recognizing and recording signs of heat. Observable signs of heat include mounting or attempting to mount other cattle, standing to be mounted by other cattle, smelling other females, trailing other females, bellowing, depressed appetite, nervous and excitable behaviour, mud on hindquarters and sides of cattle, roughed up tail hair, vulva swelling and reddening, clear vaginal mucous discharge, and mucous smeared on rump. The surest sign of heat is when a cow or heifer allows other cattle to mount her while she remains standing. This is called standing heat. Cattle may be willing to mount others but may not stand to be mounted when outside of standing heat. This usually indicates she is either coming into or going out of standing heat.

Visual observation to detect heat requires observation of cattle at least twice daily, typically early in the morning and late in the evening. More frequent observation of cattle for heat improves detection accuracy and increases the likelihood of recognizing the optimal time for breeding cattle, particularly in cattle breeds in which heat is less intense or shorter in duration. Nearly 20 per cent more cattle will be observed in heat when checked four times per day versus checking twice daily. To be effective, heat observation needs to be timed evenly over 24 hours. Each observation period must be sufficiently long, usually at least 30 minutes, to be effective, indicating that a farmer, or trained farmhand, may spend at least 2 hours daily on proper heat detection, if not helped by additional technology. Standing heat can occur any time in a 24-hour period. However, the most likely time for a cow or heifer to show heat signs is at night. The season of the year can influence this, with more cows showing heat at night in hot weather and more showing heat during the day in cold weather. Housing conditions can also have an effect on the distribution of heat during a 24-hour period. Hot weather, high production, crowded conditions, and high stress environments may reduce heat activity. Observers must distinguish among cattle coming into heat, in standing heat, and going out of heat. Females that are in standing heat, were in standing heat yesterday, or will be in standing heat tomorrow are the most likely herd mates to mount other cows or heifers in heat.

The primary objective of reproductive management for every dairy farmer is to assure cows calve at an optimal interval, resetting lactation to peak levels of efficiency. Therefore, farmers strive for a high conception rate. Conception rate (CR) is a measure of a cow's fertility at service. It is calculated by dividing the number of pregnant cows by the total number of inseminations. Respectable rates are 50 - 55%, and should be highest after the first insemination period (first service). The CR at first service is also the best indicator of the overall CR because all cows are in this calculation, including problem cows that lack conception after repeat services and may be culled later due to poor fertility. Conception rates are confounded by such factors as the physiologic fertility of the cow, overall heat detection, semen quality, and semen handling and insemination techniques. It is not an easy variable to improve, other than to make sure good semen is being properly inserted into a healthy uterus at the proper time for achieving conception. Heat detection and determining proper time of insemination are often cited as the most costly component of achieving high CR and undoubtedly, the major limiting factor to the success of AI programs on many dairy farms. Incorrect detection of estrus and missing a chance to timely inseminate is related to loss of income due to extended calving intervals, milk loss, more veterinary and heifer rearing costs and slowed genetic progress. Especially when a cow already has been inseminated earlier (i.e. after the first service) it is difficult to timely detect the need for re-insemination in case conception was not achieved and the cow is turning out to be non-pregnant after said first service. Mind that when a standing heat at 21 days after first service goes undetected, the next chance to properly inseminate in order to achieve successful conception and thus pregnancy occurs at around day 42 after first service, adding a full three weeks of less productive lag phase to the desired calving interval.

While heat detection can be a major problem for some operations, getting a female pregnant in one or two services is just as important. University dairy studies in the USA conservatively estimate the costs of prolonged calving intervals are from $3.19 to $5.41 per day per cow beyond 100 days open due to: lost milk production, decreased calf crop, increased veterinary costs, involuntary culling of non-pregnant cows and increased need for replacement cows. It is estimated that the US dairy industry loses $300 million as a result of erroneous diagnosis of and failure to predict optimal estrus. Similarly, more than 30% of the cows removed from dairy herds each year in Western Canada are culled due to reproductive problems. Further, the average 1st service conception rate to artificial insemination there is only about 40%. Based on 1 estimate, up to $230 million in potential income is lost annually by Canadian dairy farmers, because the current national average calving interval is 14 months.

When using AI the challenge of achieving an optimal calving interval depends foremost upon effective prediction of estrus. Heat detection aids are available. They are generally used to supplement but not replace visual observation. These include tail paint, tail head markers or detectors, chin-ball markers, and detection systems that record mounting or activity behaviour. Heat detection aids differ in their application method, detection method, cost per animal, and detection accuracy. Detector (teaser) animals can also assist in heat detection. Teaser animals include several types of gomer bulls, which are surgically altered to prevent successful insemination.

Over the years, science has delivered various reproduction management technologies intended to help dairy producers become more efficient and productive. For example, automated or electronic heat detection systems are showing some benefits in some herds. Using wireless technology, radio transmitters incorporating a pressure switch may for example be glued onto the tailheads of cattle to monitor mounting activity of said cattle and transmit the mounting data to a computer where software examines the mounting profile of each animal. However, much like other reproductive programs, the usefulness of automated heat detection depends on the individual dairy's management system, heat detection capabilities and breeding goals, and can also simply but severely fail when a tailhead or chin-ball heat detector or mounting or activity transmitter for example dislodges in the milking pen or when a cow is attempted to be mounted by another cow.

Attempts have been made to adapt methods that in general detect pregnancy to allow for the prediction of heat, reasoning that a cow that is positively tested to be pregnant will generally only come in heat after calving where a non-pregnant cow may generally come in heat shortly, at least within less than 3 weeks after pregnancy testing. At present, testing cows by manual trans-rectal pregnancy examination performed at 10 to 13 weeks after insemination is the most reliable method to confirm pregnancies. However, this long period after first service before a pregnancy can be detected clearly rules out use of manual trans-rectal techniques for predicting non-pregnancy and estrus at week 3 after service. Similarly, trans-rectal ultrasonography (TRUS), which is generally performed by a veterinarian to detect a budding embryo may allow detection of a pregnancy at 28-35 days, and more reliably so at 42 days, after insemination, but again fails to predict non-pregnancy and estrus at day 21 after service. Pregnancy-associated glycoproteins (PAGs) constitute a large family of glycoproteins that are synthesized in the superficial layer of the caruncles of the ruminant placenta according to a spatial and temporal expression pattern. When PAGs are released in the maternal blood they can be used for pregnancy diagnosis, pregnancy follow-up and for the monitoring of the trophoblastic function. However, embryonic implantation in ruminants starts after week 3, is non-invasive and rather slow. There only is close attachment between embryonic membranes and the endometrium overlying caruncles at 5 weeks in cattle, and only thereafter the placenta is fully established. Thus PAG measurements in blood can only be used reliably from day 35 and on after insemination or service by farmers for reproduction management. However specific for indicating pregnancy at week 5 and later, earlier (i.e. day 19) measurements of PAG are considered incapable to timely identify PAG-negative non-pregnant animals that need to be re-inseminated at week 3 from 3-week-pregnant animals that have not yet released PAG into the blood. Similarly, the Early Conception Factor (ECF) lateral flow test was evaluated for its ability to accurately determine non-pregnant status in dairy cattle. Results of 2 field trials involving 191 cows and 832 tests indicated the probability that a cow can be correctly diagnosed as non-pregnant by using the ECF test is only about 50%. Agreement of test results between milk and serum obtained from the same cow was 57.5%. The ECF test was not consistent in identifying non-pregnancy when the same cows were tested repeatedly over a period of 4 weeks. It was concluded that the ECF test does not accurately identify non-pregnancy in dairy cattle.

Progesterone (P4) measurements in blood or milk come closest to early detect pregnancy, cows showing a drop in milk P4 level below 5 ng/ml around day 21 after insemination are in general are not pregnant. However, such early P4 measurements are not at all specific, many non-pregnant animals testing solidly positive for P4 as well many days beyond day 21 because of high residual progesterone-producing activity of the corpus luteum often extending beyond day 21, even in the non-pregnant animal. Thus, in cows, a P4 test generally requires repeated testing from day 19 to 28 and is thus also not reliable for day 21 re-insemination testing, and moreover is less predictive in milk than in blood.

In short, reproductive inefficiency in dairy cattle is a major economic problem for dairy producers. If a cow is inseminated but does not conceive after a first service, she should return to heat (estrus) in approximately 21 d. However, since heat detection is less than optimal in modern dairy herds, many cows that fail to conceive are not identified until a veterinarian performs pregnancy diagnosis at about 40 days after breeding, shortly before she enters into her second heat after said service. Therefore, there is a need for a technology that allows for a sufficiently accurate determination of pregnancy or non-pregnancy at a moment in time that is less than 21 days after insemination, allowing timely re-insemination of non-pregnant cows to help to improve reproductive efficiency in dairy cattle by reducing the calving interval of those cows.

The invention

The invention provides a method to determine whether a previously inseminated female mammal is pregnant prior to a point in time at which said mammal would be expected to ovulate, and preferably prior to a point in time at which said mammal is presenting with standing heat if non-pregnant, said method comprising taking a biological sample from said mammal prior to said point in time and testing said sample for the presence of a multiple pregnancy marker pattern (MPMP). The inventors utilise herein the fact that the complex physiological processes of fertilization leading to implantation of an early embryo in the uterus followed by the development of a placenta and further development of the embryo requires the ordered expression of many proteins and other biomarkers in distinct patterns. These patterns are changing with time and establishment of pregnancy or failure of establishment of pregnancy. Failure of the expression of a single protein or hormone at the right place and the right moment, and thus disruption of this pattern, may interrupt the pregnancy and lead to an ovulation. The inventors set out to develop a test that measures the multiple patterns of pregnancy biomarkers (e.g. hormones or proteins) involved in either correct embryo development or failure of this and developed a very accurate and timely method capable of measuring the status of the multiple biological pattern processes at around the point in time that the female mammal is either expected to continue its pregnancy, or, when pregnancy has failed, expected to prepare for another ovulation. Such multiple pregnancy marker pattern (MPMP) testing can in principle be applied to a mammal of all species where there is a distinct pattern of differences to be found in pregnant versus non-pregnant mammals at around the time that said mammal would be expected to either continue its pregnancy, or prepare for an ovulation if not pregnant. Modern day proteomic analyses, preferably using mass spectrometry, are very well equipped to detect marker patterns. In principle, it is even not necessary to determine the absolute quantity of the markers involved, it suffices to record the relative different amounts of the markers under study, to provide the profile or pattern of peaks of the markers that relate to pregnancy or non-pregnancy. When a set of markers is already known, alternative testing may be contemplated with for example immunoassays developed for detecting said markers, using antibodies specifically directed against individual markers of the MPMP involved. To match for the multiple testing formats allowed by mass spectrometry, multiplex immunoassays formats might be used. Using this general principle of detecting marker patterns the inventors developed various tests to determine MPMP in cows, the results of which can be interpreted by farmers for dairy cow management. In a preferred embodiment of the invention a method is provided to determine whether a previously inseminated female mammal is pregnant prior to a point in time at which said mammal would be expected to ovulate if non-pregnant, said method comprising taking a biological sample from said mammal prior to said point in time and testing said sample for the presence of a multiple pregnancy marker pattern (MPMP), wherein said sample is tested for the relative expression of progesterone and of at least one marker selected from a group of marker proteins that each contribute to a significantly differential marker expression pattern related to the status pregnancy. It is preferred that said sample is tested to determine whether said mammal is non-pregnant and may be re-inseminated with the purpose to establish pregnancy. Herewith, the invention provides a method to determine whether an inseminated female mammal is pregnant prior to a point in time at which said mammal, according to its general ovulatory cycle of its species, is or would be expected to ovulate if non-pregnant. Said method comprising taking a biological sample from said mammal at said first point in time and testing said sample for the relative expression of progesterone and of at least one marker selected from a group of marker proteins that each have a significantly differential expression level related to the pregnancy status. The invention also provides a method to test for pregnancy or non-pregnancy of a cow at a moment in time of 21 days or less than 21 days after insemination comprising testing a sample of said cow for the relative presence of one or more proteins selected from the group of proteins consisting of kappa-casein (CSN3; herein also called MS147), Rho-related GTP-binding protein (RhoB; MFGM661), Protein disulfide-isomerase (P4HB; MS9), Arachidonate 12-lipoxygenase (ALOX12; MFGM197), Cathepsin Z (CTSZ; MS92), Osteoclast-stimulating factor 1 (OSTF1; MFGM713) and UBX domain protein 4 (UBXN4; MFGM660). This group consists of 7 proteins which were either significantly up- or significantly down-regulated as found in milk samples taken at day 19 after insemination from a group of 30 pregnant cows in comparison with milk samples from a group of 30 non-pregnant cows taken at day 19 after insemination (as studied by a T-test analysis with P<0.05 significance level). The current inventors understood that accurate pregnancy prediction of cows at an early stage prior to day 21 after service is important for dairy farmers so that they can benefit from inseminating said cow immediately at the upcoming ovulation at around 21 days instead of having to wait a full ovulatory cycle to only be able to inseminate at 42 days. Currently the earliest reliable pregnancy tests are available for testing at 35 days after insemination. Ovulation occurs at an interval of 21 days. Therefore, a pregnancy/non-pregnancy test performed prior to ovulation at 21 days, e.g. a test around 19 days after insemination is developed. Knowing that a cow is not pregnant before the next heat will make farmers aware of an upcoming heat so they can keep a better eye on the cow and act accordingly to timely inseminate it when it comes in heat. This reduces the calving interval of some cows with 21 days. Additionally, knowing that the cow is pregnant will prevent unnecessary re-insemination. This will save insemination costs, but more importantly will prevent damage to the zygote, which could lead to early embryonic death. The invention provides a method to test for pregnancy or non-pregnancy of a cow. The inventors have set out to detect proteins involved in either correct embryo development or failure that act around the time of establishing pregnancy to find biomarkers measuring the status of the biological processes involved in establishing pregnancies in cows. The inventors then developed an interpretation of tests results that is useful to be applied in reproductive dairy cow management by a farmer. Due to the major physiological changes in the cow during both a developing pregnancy and the reversion of an unsuccessful pregnancy it was understood by the inventors that blood contains components that provide a detailed and specific indication of these processes, and therefore reflect gestational status of a pregnant mammal. Circulating proteins involved in pregnancy-related processes could be potential biomarkers to detect these processes real time. Milk also contains many of the proteins circulating in blood making milk an ideal source to measure pregnancy because of its easy availability. Proteomics methodology by mass spectrometry enables a fast and wide screening of all proteins in a biological sample to detect marker proteins with variable expression related to a biological status such as pregnancy. Testing can be done on most biological samples that reflect the protein profile of blood, such as serum or plasma or in some cases urine, however, for ease of availability it is preferred that said sample is milk.

In a preferred embodiment, the invention provides a method to determine whether an inseminated female mammal is pregnant prior to a point in time at which said mammal would be expected to ovulate if non-pregnant, said method comprising taking a biological sample from said mammal prior to said point in time and testing said sample for the relative expression of progesterone and of at least one marker selected from a group of marker proteins that each have a significantly differential expression level related to the status pregnancy. It is preferred that said marker selected from said group is identified with a false discovery rate of less than 1%. It is further preferred that said expression of said at least one marker is generally detectable in biological samples of female mammals of the species tested. Moreover, a method is preferred wherein testing the relative expression of at least one marker selected from said group of biomarker proteins increases the predictive value of determining pregnancy by testing the relative expression of progesterone of said sample. To determine whether a mammal is pregnant or not the profile or pattern of the relative expressions of the proteins and the progesterone is calculated. To develop the algorithm or formula to assess outcome of results, it is preferred to use an all-possible-regressions method and calculate cross-validated estimates of specificity, sensitivity and percentage correctly classified using generally available statistical software, which ads the advantage that repeat calculations with increased sets of data from said distinct species of mammals tested improves on the formula for the specific species under study.

In some embodiments of the invention a method is provided wherein testing said sample is done by mass spectrometry. This is an analytical chemistry technique wherein the mass-to-charge ratio of proteins can be determined. Using mass spectrometry, proteins are ionized in order to obtain charged proteins and/or charged protein fragments. These charged proteins and/or fragments are then typically accelerated and subjected to an electric or magnetic field. The amount of deflection of the charged proteins and fragments is indicative for their mass-to-charge ratio. In a preferred embodiment, said mass spectrometry comprises liquid chromatography- mass spectrometry (LC-MS) or multiple reaction monitoring (MRM). In a most preferred embodiment, LC-MS/MS technology is used to test said sample. LC-MS/MS technology as further detailed in the examples in this application herein provides the required multiple pregnancy marker pattern recognition and predict proper insemination time as provided by the invention herein.

In some other embodiments of the invention a method is provided wherein testing said sample is done by immunoassay. In preferred embodiments, said immunoassay comprises an enzyme-linked immunosorbent assay (ELISA), such as is for example used for progesterone (P4) testing, or a radio-immunoassay (RIA), and/or a Western Blot assay, provided with a binding compound useful in immunoassays, such as a specific antibody. In some embodiments, a dipstick ELISA is performed. Preferably, a dipstick is provided with a binding compound useful in immunoassays. In a much preferred embodiment, said immunoassay comprises a multiplex immunoassay to simultaneously measure multiple specific protein targets in a single sample, for example based on Luminex® xMAP® technology. Such a multiplex assay may also provide the multiple pregnancy marker pattern recognition and predict proper insemination time as provided by the invention herein. Antibodies useful in such immunoassays are available (see Table 7) or can be made in the art. Testing can be done on most biological samples that reflect the protein profile of blood, such as serum or plasma, urine or in some cases milk. Preferably the kind of biological sample that is easy and non-invasive to collect (e.g. milk for dairy cows, urine for humans). The invention may find application in various animal species or even in humans. Care needs to be taken to adjust the time point of testing to the respective ovulatory cycle of the species under study. Humans have an ovulatory cycle of 28 days and typically do not present standing heat as other animals may do. In one embodiment a method to determine whether an inseminated female human is pregnant prior to a point in time at which said mammal is, according to its 28-day general ovulatory cycle of its species, is or would be expected to ovulate if non-pregnant, said method comprising taking a biological sample from said female at said first point in time and testing said sample for the relative expression of progesterone and of at least one marker selected from a group of marker proteins that each have a significantly differential expression level related to the status pregnancy in humans, in particular to provide multiple pregnancy marker pattern recognition to predict proper insemination time in a human.

Animals that present with standing heat prior to ovulation are typically found among Perissodactyla, in particular horses or donkeys, and among Artiodactyla, in particular cows, goats en sheep, and pigs. Goats having an ovulatory cycle of 19 days, in one other embodiment a method to determine whether an inseminated female goat is pregnant prior to a point in time at which said goat is, according to its 19-day general ovulatory cycle of its species, is or would be expected to ovulate if non-pregnant, said method comprising taking a biological sample from said female goat at said first point in time and testing said sample for the relative expression of progesterone and of at least one marker selected from a group of marker proteins that each have a significantly differential expression level related to the status pregnancy in goats in particular to provide multiple pregnancy marker pattern recognition to predict proper insemination time in a goat.

Sheep having an ovulatory cycle of 19 days, in one other embodiment a method to determine whether an inseminated female sheep or ewe is pregnant prior to a point in time at which said sheep is, according to its 19-day general ovulatory cycle of its species, is or would be expected to ovulate if non-pregnant, said method comprising taking a biological sample from said ewe at said first point in time and testing said sample for the relative expression of progesterone and of at least one marker selected from a group of marker proteins that each have a significantly differential expression level related to the status pregnancy in sheep, in particular to provide multiple pregnancy marker pattern recognition to predict proper insemination time in a ewe.

Pigs having an ovulatory cycle of 21 days, in one other embodiment a method to determine whether an inseminated female pig or sow is pregnant prior to a point in time at which said sow is, according to its 21-day general ovulatory cycle of its species, is or would be expected to ovulate if non-pregnant, said method comprising taking a biological sample from said sow at said first point in time and testing said sample for the relative expression of progesterone and of at least one marker selected from a group of marker proteins that each have a significantly differential expression level related to the status pregnancy in sows, in particular to provide multiple pregnancy marker pattern recognition to predict proper insemination time in a sow.

Horses having an ovulatory cycle of 21 days, in one other embodiment a method to determine whether an inseminated female horse or mare is pregnant prior to a point in time at which said mare is, according to its 21-day general ovulatory cycle of its species, is or would be expected to ovulate if non-pregnant, said method comprising taking a biological sample from said mare at said first point in time and testing said sample for the relative expression of progesterone and of at least one marker selected from a group of marker proteins that each have a significantly differential expression level related to the status pregnancy in mares, in particular to provide multiple pregnancy marker pattern recognition to predict proper insemination time in a mare.

Cows having an ovulatory cycle of 21 days, in one other embodiment a method to determine whether an inseminated cow is pregnant prior to a point in time at which said cow is, according to its 21-day general ovulatory cycle of its species, is or would be expected to ovulate if non-pregnant, said method comprising taking a biological sample from said cow at said first point in time and testing said sample for the relative expression of progesterone and of at least one marker selected from a group of marker proteins that each have a significantly differential expression level related to the status pregnancy in cows, in particular to provide multiple pregnancy marker pattern recognition to predict proper insemination time in a cow. Individual cows having sometimes a somewhat shorter (i.e as short as 19-20 days) or a somewhat longer (i.e. as long as 22-23 days) ovulatory cycle, it is preferred that said sample is taken from 17 - 21 days, preferably from 18 - 20 days, more preferably at 19 days after insemination of said cow to best predict proper re-insemination time. In particular a method to test in cows is provided wherein said group of marker proteins comprises kappa-casein (CSN3), Rho-related GTP-binding protein (RhoB), Protein disulfide-isomerase (P4HB), Arachidonate 12-lipoxygenase (ALOX12), Cathepsin Z (CTSZ), Osteoclast-stimulating factor 1 (OSTF1) and UBX domain protein 4 (UBXN4). When testing in cows, it is preferred that said group of at least one marker comprises UBXN4. As shown in the detailed description herein, when the progesterone concentration in a cow's milk sample is higher than 5 ng/ml and the UBXN4 concentration in a milk sample is determined and the formula Y = (-17.8933+(2.4546*UBXN4)+(0.1472*Progesterone) is applied, where Y>0 the animal is deemed pregnant. If Y<0 the animal is deemed not pregnant. The mean sensitivity of this determination based on testing the 60 cows as described in the specification herein is 82%, mean specificity is 80%, and 81% correctly classified When testing in cows it is even more preferred that said group of at least one marker comprises the two markers CSN3 and P4HB. As shown in the detailed description herein, when the progesterone concentration in said milk sample is higher than 5 ng/ml, and the kappa-casein concentration and the Protein-disulfide-isomerase concentration in said milk sample are determined and the formula Y = (75.5618+(-2.7697*CSN3)+(-9.7142*P4HB)+(0.1707*Progesterone) is applied, where Y>0 the animal is deemed pregnant. If Y<0 the animal is deemed not pregnant. The mean sensitivity of this determination based on testing the 60 cows as described in the specification herein is 86%, mean specificity is 85%, and 85% correctly classified.

When testing in cows it is even more preferred that said group of at least one marker comprises the three markers CSN3, P4HB and OSTF1. As shown in the detailed description herein, when the progesterone concentration in said milk sample is higher than 5 ng/ml, and the kappa-casein concentration, the Protein-disulfide-isomerase concentration, and the Osteoclast-stimulating factor 1 concentration in said milk sample are determined and the formula Y = (86.6024+(-3.3000*CSN3)+(-14.9385*P4HB)+(3.3527*OSTF1)+ (0.2242*Progesterone), is applied, where Y>0 the animal is deemed pregnant. If Y<0 the animal is deemed not pregnant. The mean sensitivity of this determination based on testing the 60 cows as described in the specification herein is 92%, mean specificity is 93%, and 92% correctly classified.

When testing in cows it is even more preferred that said group of at least one marker comprises the four markers CSN3, P4HB, RhoB, and ALOX12. As shown in the detailed description herein, when the progesterone concentration in said milk sample is higher than 5 ng/ml, and the kappa-casein concentration, the Rho-related GTP-binding protein concentration, the Protein-disulfide-isomerase concentration, and the Arachidonate 12-lipoxygenase, concentration in said milk sample are determined and the formula Y = (8954.89+(-365.97*CSN3)+ (1517.26*P4HB)+(217.79*RhoB)+ (166.7*ALOX12)+(18.81*Progesterone) is applied, where Y>0 the animal is deemed pregnant. If Y<0 the animal is deemed not pregnant. The mean sensitivity of this determination based on testing the 60 cows as described in the specification herein is 96%, mean specificity is 93%, and 94% correctly classified.

When testing in cows it is even more preferred that said group of at least one marker comprises the five markers CSN3, P4HB, RhoB, ALOX12 and CTSZ. As shown in the detailed description herein, when the progesterone concentration in said milk sample is higher than 5 ng/ml, and the kappa-casein concentration, the Rho-related GTP-binding protein concentration, the Protein-disulfide-isomerase concentration, the Arachidonate 12-lipoxygenase, concentration, and the Cathepsin Z concentration in said milk sample are determined, and the formula Y = (6163.87 + (-247.94*CSN3) + (-969.05*P4HB) + (100.93*RhoB) + (100.38*ALOX12) + (-17.25*CTSZ) + (15.49*Progesterone) is applied, where Y>0 the animal is deemed pregnant. If Y<0 the animal is deemed not pregnant. The mean sensitivity of this determination is determination based on testing the 60 cows as described in the specification herein 96%, mean specificity is 96%, and 96% correctly classified. Kits of parts comprising detection means for one or more markers according to the invention are also provided herewith. Such kits of parts are particularly suitable for use in typing milk samples. Further provided is, therefore, a kit of parts comprising detection means of one or more markers selected from the group consisting of kappa-casein (CSN3), Rho-related GTP-binding protein (RhoB), Protein disulfide-isomerase (P4HB), Arachidonate 12-lipoxygenase, (ALOX12), Cathepsin Z (CTSZ), Osteoclast-stimulating factor 1 (OSTF1) and UBX domain protein 4 (UBXN4). In some embodiments, a kit of parts according to the invention also comprises detection means of progesterone. This is, however, not necessary because commercially available progesterone testing kits can also be used in combination with one or more kits of parts according to the present invention. As described herein before, said detection means preferably comprise one or more binding compounds, preferably one or more antibodies or functional equivalents or functional parts thereof, capable of specifically binding one or more marker proteins according to the invention. These kits of parts are particularly suitable for detecting and/or quantifying marker proteins that are present in milk samples.

In one preferred embodiment, a kit of parts is provided that comprises a binding compound, preferably an antibody or a functional equivalent or functional part thereof, that is capable of specifically binding UBX domain protein 4. Such kit of parts is particularly suitable for typing bovine milk samples in order to test for pregnancy. As explained herein before, when combined with detection means for progesterone, pregnancy can be determined with a mean sensitivity of 0.82 and a mean specificity of 0.80, which are higher than the mean sensitivity and the mean specificity, respectively, of currently used milk progesterone tests. In one preferred embodiment, a kit of parts according to the invention comprising detection means for UBX domain protein 4 also comprises detecting means for progesterone. This is, however, not necessary since commercially available progesterone testing kit can also be used in combination with a kit of parts according to the invention comprising detection means for UBX domain protein 4.

In one preferred embodiment, a kit of parts is provided that comprises:

- a binding compound, preferably an antibody or a functional equivalent or functional part thereof, that is capable of specifically binding kappa-casein, and

- a binding compound, preferably an antibody or a functional equivalent or functional part thereof, that is capable of specifically binding Protein-disulfide-isomerase.

Such kit of parts is particularly suitable for typing bovine milk samples in order to test for pregnancy. As explained herein before, when combined with detection means for progesterone, pregnancy can be determined with a mean sensitivity of 0.86 and a mean specificity of 0.85, which are higher than the mean sensitivity and the mean specificity, respectively, of currently used milk progesterone tests. In one preferred embodiment, a kit of parts according to the invention comprising detection means for kappa-casein and Protein-disulfide-isomerase also comprises detection means for progesterone. This is, however, not necessary since commercially available progesterone testing kit can also be used in combination with a kit of parts according to the invention comprising detection means for kappa-casein and Protein-disulfide-isomerase.

In one preferred embodiment, a kit of parts is provided that comprises:

- a binding compound, preferably an antibody or a functional equivalent or functional part thereof, that is capable of specifically binding kappa-casein, and
- a binding compound, preferably an antibody or a functional equivalent or functional part thereof, that is capable of specifically binding Protein-disulfide-isomerase, and
- a binding compound, preferably an antibody or a functional equivalent or functional part thereof, that is capable of specifically binding Osteoclast-stimulating factor 1.

Such kit of parts is particularly suitable for typing bovine milk samples in order to test for pregnancy. As explained herein before, when combined with detection means for progesterone, pregnancy can be determined with a mean sensitivity of 0.92 and a mean specificity of 0.93, which are higher than the mean sensitivity and the mean specificity, respectively, of currently used milk progesterone tests. In one preferred embodiment, a kit of parts according to the invention comprising detection means for kappa-casein, Protein-disulfide-isomerase and Osteoclast-stimulating factor 1 also comprises detection means for progesterone. This is, however, not necessary since commercially available progesterone testing kit can also be used in combination with a kit of parts according to the invention comprising detection means for kappa-casein, Protein-disulfide-isomerase and Osteoclast-stimulating factor 1.

In one preferred embodiment, a kit of parts is provided that comprises:

- a binding compound, preferably an antibody or a functional equivalent or functional part thereof, that is capable of specifically binding kappa-casein, and
- a binding compound, preferably an antibody or a functional equivalent or functional part thereof, that is capable of specifically binding Rho-related GTP-binding protein, and
- a binding compound, preferably an antibody or a functional equivalent or functional part thereof, that is capable of specifically binding Protein-disulfide-isomerase, and
- a binding compound, preferably an antibody or a functional equivalent or functional part thereof, that is capable of specifically binding Arachidonate 12-lipoxygenase,.

Such kit of parts is particularly suitable for typing bovine milk samples in order to test for pregnancy. As explained herein before, when combined with detection means for progesterone, pregnancy can be determined with a mean sensitivity of 0.96 and a mean specificity of 0.93, which are higher than the mean sensitivity and the mean specificity, respectively, of currently used milk progesterone tests. In one preferred embodiment, a kit of parts according to the invention comprising detection means for kappa-casein, Rho-related GTP-binding protein, Protein-disulfide-isomerase and Arachidonate 12-lipoxygenase, also comprises detection means for progesterone. This is, however, not necessary since a commercially available progesterone testing kit can also be used in combination with a kit of parts according to the invention comprising detection means for kappa-casein, Rho-related GTP-binding protein, Protein-disulfide-isomerase and Arachidonate 12-lipoxygenase.

In one preferred embodiment, a kit of parts is provided that comprises:

- a binding compound, preferably an antibody or a functional equivalent or functional part thereof, that is capable of specifically binding kappa-casein, and
- a binding compound, preferably an antibody or a functional equivalent or functional part thereof, that is capable of specifically binding Rho-related GTP-binding protein, and
- a binding compound, preferably an antibody or a functional equivalent or functional part thereof, that is capable of specifically binding Protein-disulfide-isomerase, and
- a binding compound, preferably an antibody or a functional equivalent or functional part thereof, that is capable of specifically binding Arachidonate 12-lipoxygenase, , and

- a binding compound, preferably an antibody or a functional equivalent or functional part thereof, that is capable of specifically binding Cathepsin Z.

Such kit of parts is particularly suitable for typing bovine milk samples in order to test for pregnancy. As explained herein before, when combined with detection means for progesterone, pregnancy can be determined with a mean sensitivity of 0.96 and a mean specificity of 0.96, which are higher than the mean sensitivity and the mean specificity, respectively, of currently used milk progesterone tests. In one preferred embodiment, a kit of parts according to the invention comprising detection means for kappa-casein, Rho-related GTP-binding protein, Protein-disulfide-isomerase, Arachidonate 12-lipoxygenase, and Cathepsin Z also comprises detection means for progesterone. This is, however, not necessary since a commercially available progesterone testing kit can also be used in combination with a kit of parts according to the invention comprising detection means for kappa-casein, Rho-related GTP-binding protein, Protein-disulfide-isomerase, Arachidonate 12-lipoxygenase, and Cathepsin Z. Antibodies useful in such kits-of-parts are available (see Table 7) or can be made in the art.

The detection means of a kit of parts according to the invention are preferably present on a solid carrier. Preferred solid carriers include a protein microarray, a dipstick, a strip, a chip and/or a biosensor. These solid carriers allow easy testing of milk samples.

Use of detection means of one or more markers selected from the group consisting of kappa-casein (CSN3), Rho-related GTP-binding protein (RhoB), Protein disulfide-isomerase (P4HB), Arachidonate 12-lipoxygenase, (ALOX12), Cathepsin Z (CTSZ), Osteoclast-stimulating factor 1 (OSTF1) and UBX domain protein 4 (UBXN4), for determining the presence and/or (relative) amount of one or more of said markers in a biological sample, preferably in cow milk.

Brief description of the drawing

Figure 1
Comparison of milk progesterone levels (P4) and Pregnancy Associated Glycoproteins (PAG) concentrations in milk of pregnant and non-pregnant cows at days 19 and 35 after insemination. A and B: P4 and PAG at day 19 of pregnant (A) and non-pregnant (B) cows; C and D: P4 and PAG at day 35 of pregnant (C) and non-pregnant (D) cows; E: P4 at day 19 and PAG at day 35 of pregnant cows.; F and G: P4 at day 19 and at day 35 of pregnant (F) and non-pregnant (G) cows.
Figure 2
Events and time intervals associated with standing heat and insemination in cows.

Examples

Reproduction management is an important economic factor in dairy cattle. Insemination success rate has been reduced during the last decades. As a consequence dairy cows increasingly enter the less productive late lactation phase. On the other hand farmers need to avoid costly unnecessary re-inseminations. Therefore, there is a need for a test enabling correct re-insemination decisions. The first option for re-insemination is at day 21 after insemination. Until now high efficient detection methods of pregnancy in cows before day 21 after insemination are lacking - reliable detection of pregnancy is not possible before day 35 of gestation. The objective of this research was to develop a biomarker in the milk of cows indicating the gestational status of cows 19 days after insemination. Lactating dairy cows were inseminated and pregnancy was determined at day 19 with the milk progesterone test and at day 35 using trans-rectal ultrasonography. We studied milk proteome expression profiles of 30 pregnant and 30 non-pregnant cows 19 days after insemination. The results were analyzed with the "all possible regressions" method and the biomarkers were validated with the "leave-one-out cross validation" method. The best biomarker was a combination of a maximum of five different proteins plus Progesterone showing 96% predictive capacity. Functional annotation analysis of the ten proteins in the three best biomarker combinations revealed that eight of them are expressed in either the embryo or the placenta; the other two are expressed in the mammary gland. We conclude that our biomarker group has the potential to indicate the non-pregnant cows for re-insemination at day 19. A milk-pregnancy test could be performed on day 19 milk samples as a 'cow-side' test, or could be incorporated in a milking robot environment.

Reproduction management is an important economic factor in dairy cattle. A short calving interval is important for maintaining high productivity. During the last decades the calving interval increased (Lucy, 2001). Early pregnancy detection is an important tool for reproduction management. Due to poor estrus expression of cows that return to cycling after insemination the farmer may not detect the estrus (Yoshida and Nakao, 2005). As a consequence dairy cows increasingly enter the less productive late lactation phase. On the other hand farmers need to avoid unnecessary re-inseminations, which are costly and endanger abortion. Especially during the early gestation the recognition of pregnancy, failure to become pregnant, or the loss of a very small early embryo are difficult to detect. At present reliable pregnancy detection is not possible before day 35 of the pregnancy. For efficient re-insemination at the earliest possible moment, i.e. 21 days after the insemination, it is necessary to discriminate the pregnant cows from the non-pregnant ones before day 21. Therefore, there is a need for a test enabling correct re-insemination decisions. Improved testing methods could have economic merit for dairy farmers if the costs of testing are lower than the losses caused by the missing benefits of late recognition of pregnancy failure. At present milk or blood progesterone measurements, trans-rectal ultrasonography (TRUS) or hand feeling, and PAG (Pregnancy Associated Glycoproteins, Szenci et al., 1998) measurements at day 35 after insemination are used by farmers for reproduction management. However, the progesterone test requires repeated testing from day 19 to 28 and is less predictive in milk than in blood (Gillis et al., 2006) and is thus not reliable for day 21 re-insemination. Also the PAG test detects proteins at day 35 after insemination, and is not reliable at day 19. Similarly, other approaches to detect early pregnancy using single factors such as Early Conception Factor failed due to high false positive or false negative prediction rates (Cordoba et al., 2001; Ambrose et al., 2007). The complex physiological processes of fertilization leading to implantation of an early embryo in the uterus followed by the development of a placenta and further development of the embryo requires the ordered expression of many proteins (Memili and First, 1999). Failure of the expression of a single protein at the right place and the right moment may interrupt the pregnancy. If a test measures the proteins involved in either correct embryo development or failure of this it will be a very accurate and timely biomarker measuring the status of the biological processes. Such a test can be interpreted for dairy cow management of the farmer.

Due to the major physiological changes in the cow during both a developing pregnancy and the reversion of an unsuccessful pregnancy it may be expected that the blood circulation will contain components that provide a detailed and specific indication of these processes, and therefore of the gestational status of the cow. Circulating proteins involved in pregnancy-related processes could be potential biomarkers to detect these processes real time. Milk also contains many of the circulating proteins (Petit, 2003) making milk an ideal source to measure pregnancy.

Proteomics methodology enables to perform a wide screening of all proteins in a biological sample and detect the proteins with variable expression related to a biological status such as pregnancy. The objective of this research was to identify proteins in the milk of inseminated cows enabling to develop a biomarker in the milk of cows indicating the pregnancy status of cows 19 days after insemination. The final biomarker can be a combination of molecules including proteins.

Animals, experimental design, and milk collection and analyses

Milk samples from lactating cows were collected on days 19, 28 and 35 after insemination on nine Dutch farms in the period mid-July to mid-September 2012. Progesterone concentration in the milk samples was determined as described below. The cows were monitored for return to heat in the weeks following insemination. Cows that did not return to heat until day 35 were tested for pregnancy by trans-rectal ultrasonography (TRUS) on day 35-38. For studying associations of proteomics data with pregnancy/non-pregnancy, cows that were diagnosed pregnant on day 35 by TRUS (which all had milk progesterone values higher than 9.5 ng/ml on each of days 19, 28, and 35) were defined as pregnant. Cows that returned to heat (all except one were re-inseminated), and had a milk progesterone value lower than 5 ng/ml on day 19, or on day 28 were defined as non-pregnant. Day 19 milk samples from 30 pregnant and 30 non-pregnant cows were used for the proteomics analyses. The number of cows used in the study varied between farms, but pregnant and non-pregnant cows were balanced per farm (Table 1). For comparison, we have also used the day 19 and day 35 milk samples for a commercial bovine milk pregnancy test based on Pregnancy Associated Glycoproteins (PAG) (Szenci et al., 1998) (which is intended for day 35 milk samples).

Proteomics and bioinformatics

Milk serum (Milks) and cream (containing the Milk Fat Globule Membrane (MFGM)) fractions were separated by centrifugation for 20 minutes at 1500 x g at 4°C and separately collected. The milk serum fraction was cleared by centrifugation for 90 min. at 100,000 x g. Fractions were stored at -80°C until use. The milk cream fraction was washed four times with water and centrifuged at 1500 x g. After the final wash, the cream fraction was diluted with an equal volume of 4% SDS in 100 mM TRIS/HCl (pH 7,6) and sonicated for 4 min. in a water bath sonicator. After centrifugation for 10 min. at 1500 x g the supernatant (i.e. the MFGM fraction containing the fat globule proteins) was collected and stored at -80°C until use (Lu et al., 2011).

Nano LC Fourier transform MSMS (FTMS) label free analyses were performed (LC-MS/MS; Lu et al., 2011; Smaczniak et al., 2012) to identify the differentially or variable expressed proteins. Full scan positive mode FTMS spectra were measured between mass-to-charge ratios of 380 and 1400 with a LTQ-Orbitrap XL mass spectrometer (Thermo electron, San Jose, CA, USA) at high resolution (60000). CID MSMS scans of the four most abundant multiply charged peaks in the FTMS scan were recorded in data dependent mode in the linear trap (MSMS threshold = 5.000).

LCMS runs with all MSMS spectra obtained were analyzed with MaxQuant 1.3.0.5 (Cox and Mann 2008, Smaczniak et al., 2012) using default settings for the Andromeda search engine (Cox et al., 2011) except that extra variable modifications were set for de-amidation of N and Q. A Bos taurus database downloaded from Uniprot (http://www.uniprot.org) was used together with a contaminants database that contains 60 sequences of common contaminants as for instance: Trypsin (P00760, bovine), Trypsin (P00761, porcine), Keratin K22E (P35908, human), Keratin K1C9 (P35527, human), Keratin K2C1 (P04264, human) and Keratin K1CI (P35527, human). For protein identification, peptides and proteins with a false discovery rate (FDR) of less than 1% and proteins with at least 2 identified peptides one or more of which were unique and at least one unmodified, were accepted. Filtering and further bioinformatic analysis of the MaxQuant/Andromeda workflow output and the analysis of the ratios of the identified proteins were performed with the Perseus 1.3 module (available at the MaxQuant suite). Reversed hits were deleted from the MaxQuant result table. Up- and down-regulated proteins were investigated more detailed using the UNIPROT (http://www.uniprot.org/), Gene Ontology (http://www.geneontology.org/) and KEGG (http://www.genome.jp/kegg/) websites to obtain the biological functions of the proteins to evaluate their biological activities in relation to presence or absence of a pregnancy. Expressions in embryo or placenta were investigated using Expression Atlas (http://www.ebi.ac.uk/gxa/home).

Statistical analysis-in relation with bioinformatics

Only proteins with detectable expression levels in all animals and thus were generally detectable in all animals tested were used for the statistical analysis. This eliminated the proteins with very low expression levels. A statistical screening was performed to evaluate the relationship between the dependent variable pregnancy outcome and the independent variables protein abundances and milk progesterone (P4) levels. The goodness of fit was assessed by two different statistics, the mean deviance and the Pearson chi-square statistics. A P-value for the chi-square test less than 0.05 was considered statistically significant. The proteins s that had significantly differential expression levels in pregnant versus non-pregnant cows were used for further analysis.

In order to select the best subset of predictor variables from a larger set we used the "all-possible-regressions" method with a maximum of six independent variables. This method tests literally all possible subsets with one to six independent variables of the set of potential independent variable. Analyses were performed using logistic regression model for binomial data (generalized linear models, GLM, family = binomial, link = logistic) using the GLM function in R (R Core team, 2014). In order to evaluate the performance of each model we performed a leave-one-out cross validation (LOOCV) using the cross.val function of the R package "R330" (Lee et al., 2012). LOOCV involves using a single observation from the whole dataset as the validation set, and the remaining observations as the training set; the process is repeated such that each observation in the dataset is used once as a validation set. For a logistic model, the function cross.val calculates cross-validated estimates of specificity, sensitivity and percentage correctly classified.

Progesterone levels in the milk at day 19 after insemination

Table 2 shows the progesterone levels in the milk. As expected, progesterone was higher in pregnant cows as compared with non-pregnant cows. Due to the variation of the progesterone levels the milk progesterone levels cannot separate the pregnant and non-pregnant cows.

Progesterone (P4) and Pregnancy Associated Glycoproteins (PAG) at day19 after insemination

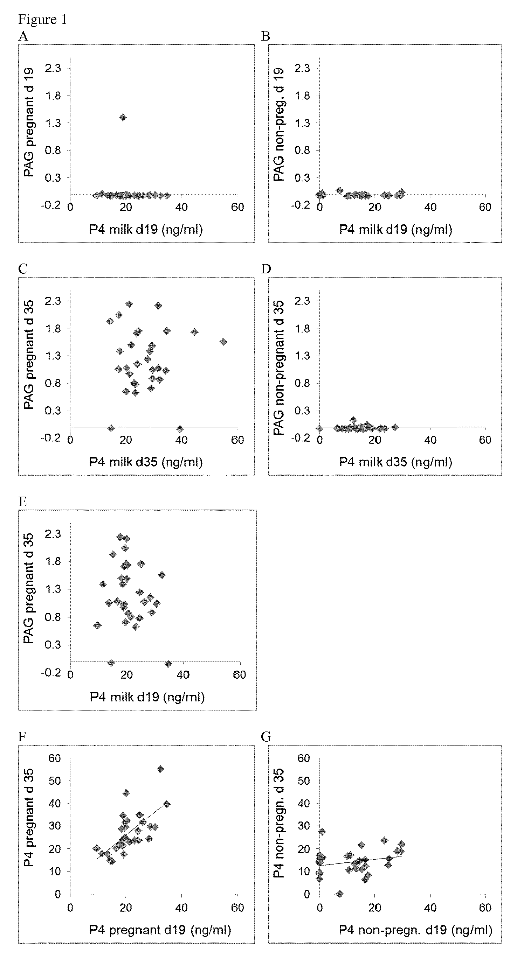

Figure 1 shows a comparison between milk progesterone and PAG levels in the milk of cows 19 and 35 days after insemination. In milk of day 19, PAG levels were low in all pregnant cows but one, confirming that PAG cannot be used to diagnose pregnancy on day 19. In day 35 milk, PAG levels agreed with the d35 TRUS outcome in most cows, showing high levels in all but 2 pregnant cows (sensitivity of 93%) and low in all non-pregnant cows (Figure 1B) specificity 100%). In non-pregnant cows, the PAG concentrations were always low while the progesterone levels vary.

Proteomics measurements

The milk proteomes of the milk serum and the MFGM fractions were separately determined. The proteomics measurements identified 81 milk serum proteins and 253 MFGM proteins, together 334 proteins (some proteins were detected in both fractions) detected in all samples. About the same number of proteins were detectable in the milk of some cows, while the expression levels were below detection threshold in the milk of other cows. All of the proteins were identified and quantified by at least 2 peptides through comparative analysis with databases. These identifications were used in the bioinformatic analyses following the statistical analysis.

Statistical analysis

All milk protein data and milk progesterone levels on day 19 were investigated for association with pregnancy or non-pregnancy determined on day 35. Statistical association analysis revealed 27 proteins showing association with the gestational status of the cows. The expression levels of the proteins varied between highly abundant to low. An example of the results found can be found in Table 6. Table 3 summarizes the result of the statistical analysis. Progesterone was the most predictive single biomarker in terms of percentage correctly classified cows (Table 3). Adding proteins improves the predictive capacity of the combinations of biomarkers (Table 3). The number of correctly classified cows reached 96 percent for a combination of progesterone and five proteins. Both the sensitivity and the specificity of this biomarker were also 96 percent. Table 3 shows the best biomarker combinations with 1-6 biomarkers, respectively. Progesterone was included in all. The MFGM660 protein was the first protein included in a biomarker (composed of two components), but was not retained as a component of biomarkers composed of three to six components. Other proteins such as MS147, MS9 and MFGM661 were recurring components of subsequent biomarker combinations. The biomarkers composed of five and six components show high similarity, suggesting that a stabilization of the biomarker composition occurred.

Biological analysis of the proteins

The identification of the proteins was used to investigate the functional biological annotation of the proteins in the biomarker combinations using databases. Table 4 shows that five of the proteins are expressed in the embryo or the placenta, indicating involvement in pregnancy. More specifically, ALOX12 is known to be expressed pre-/postpartum, which proves that it is involved in pregnancy. However, relation with early pregnancy was not found in the database. Two proteins were annotated showing expression in the mammary gland.

The objective of this research was to identify cow milk proteins associated with pregnancy enabling to develop a biomarker for lactating inseminated cows to detect pregnancy at day 19 after insemination. Farmers can use such a pregnancy test to improve the reproduction management of their cows. Since pregnancy impact the cows physiology it is expected that the proteome of the circulation - and thereby the proteome of the milk - will show the status of the pregnancy. Previously, we showed that biomarkers to monitor and predict several traits can be developed using proteomics technologies (te Pas et al., 2013a, b). Here we show that proteomics technologies can be used to develop biomarkers to monitor the cow's pregnancy status at day 19 after insemination.

Levels of Progesterone and Pregnancy Associated Glycoprotein (PAG) in the milk at day 19 after insemination

A milk progesterone level lower than 5 ng/ml is considered to be an indication of non-pregnancy (Bulman and Lamming, 1978; Osman et al., 2012). However, milk progesterone levels on day 19 higher than 5 ng/ml can relate to both pregnancy and non-pregnancy, as non-pregnant cows on day 19 may still be in the luteal phase and hence have high progesterone levels. Pregnancy testing by TRUS was done on day 35 after insemination. Pregnant cows on day 35 were also pregnant on day 19. But cows that returned to heat before day 35 and cows that were seen as not pregnant on day 35 by TRUS may have been pregnant on day 19, but with an embryonic loss after day19. Therefore, only cows that returned to heat and had a milk progesterone value lower than 5 ng/ml on day 19, or on day 28 were defined as non-pregnant. Even then, it is possible that a cow that had low progesterone levels on day 28 but not on day 19 had been pregnant on day 19, but aborted between day 19-28. However, it is not possible by measurement of progesterone (or by any other means) to discriminate pregnant cows with early embryonic death (before day 28) from cows with a long luteal phase. Although less than in the period before day 19, some embryonic losses may occur in the period between day 19 and 28 (Vasconcelos et al., 1997; Lucy, 2001; Silke et al., 2002). The presence of such cows in our 'non-pregnant' group can only lead to an underestimation, not an overestimation, of the specificity, because animals correctly identified as pregnant on day 19 on the basis of the milk proteins, but defined as 'non-pregnant' on the basis of low progesterone levels on day 28 will be incorrectly counted as 'false positives'.

Our results also clearly indicate that the PAG test developed to detect pregnancy at day 35 after insemination was unable to detect pregnancy at day 19 after insemination. PAG are placental proteins not expressed before day 25 of pregnancy (Green et al., 2000). Therefore, we could not use the PAG test as a "gold-standard" for pregnancy in our experiments. Because neither of the existing biomarkers or tests for bovine pregnancy is able to predict pregnancy before the first next possible heat, earlier biomarkers are needed for early pregnancy diagnosis and optimal reproduction management of lactating dairy cows.

Proteomics biomarker development

Statistical association analysis revealed 27 proteins showing association with the gestational status of the cows.

Although several of the proteins showed relative low expression as compared to other non-associating proteins, all proteins could be detected in all samples. Proteins that showed expression levels under the detection limit were not used for association analysis. Therefore, the expression level is not expected to affect the results of the statistical association analysis.

While each of the 27 proteins showing association with the gestational status of the cows had a limited predictive capacity, the combination of progesterone plus five proteins showed a very high predictive capacity. For example, the MFGM660 protein (UBX domain-containing protein 4 (UBXN4)-an integral endoplasmic reticulum membrane protein that promotes the degradation of ER-associated proteins - has a low predictive capacity as it only correctly classified 52% of the samples. Remarkably however, the protein has the highest predictive capacity in the biomarker combination composed of 2 components - together with progesterone. This suggests that the functions of MFGM660 and progesterone are highly complementary. Together they cover a wider spectrum of the pregnant - non-pregnant diversity than any other combination of two components. Adding more components removes MFGM660 from the best biomarker combination. This suggests that these proteins together cover a wider spectrum of the pregnant - non-pregnant diversity than MFGM660. It has been shown before that adding more proteins to a biomarker often improves the prediction and reliability (te Pas et al., 2013b). However, it should be noted that this biomarker awaits an independent validation - i.e. with an independent study and dataset derived from another herd, or at least other cows-to validate the high predictive capacity for general applicability.

How to determine pregnancy after the biomarker measurements in cows based on the MPMP results obtained herein. Table 5 shows that cutoff values for each individual factor may not be very informative and the combination of factors including calculation with the formula are essentially improving tests.

When the MPMP biomarker consists of 5 proteins and progesterone. After quantitative measuring these 6 factors, pregnant or non-pregnant should be determined using the formula:

6 variables

$$Y = (6163.87 + (-247.94*CSN3) + (-969.05*P4HB) + (100.93*RhoB) + (100.38*ALOX12) + (-17.25*CTSZ) + (15.49*Progesterone)$$

If Y>0, than the cow is pregnant.

5 variables

$$Y = (8954.89+(-365.97*CSN3)+(1517.26*P4HB)+(217.79*RhoB)+(166.7*ALOX12)+(18.81*Progesterone)$$

If Y>0, than the cow is pregnant.

4 variables

$$Y = (86.6024+(-3.3000*CSN3)+(-14.9385*P4HB)+(3.3527*OSTF1)+(0.2242*Progesterone)$$

If Y>0, than the cow is pregnant.

3 variables

$$Y = (75.5618 + (-2.7697*CSN3) + (-9.7142*P4HB) + (0.1707*Progesterone)$$

If Y>0, than the cow is pregnant.

2 variables

$$Y = (-17.8933 + (2.4546*UBX) + (0.1472*Progesterone)$$

If Y>0, than the cow is pregnant.

1 variable

$$Y = (-2.3280 + (0.1389*Progesterone)$$

If Y>0, than the cow is pregnant.

Adding biological information of the associating proteins to the statistical results enables to develop a relevant biological mechanism for a number of proteins. This often enhances the reassurance of the results (te Pas et al., 2013a, b). Unfortunately the annotation of many genes / proteins is still lacking, or at best unfinished (Stelzl et al., 2005). Thus, it can be expected that relevant proteins will be lost by this method. The best biomarker combinations contained proteins that could be related to pregnancy (expression known to be in embryo or placenta (http://www.ebi.ac.uk/gxa/home)). Therefore, the biological analysis strengthen the conclusion that this biomarker detects early pregnancy (Balhara et al., 2013).

Relevance for the dairy industry

The present MPMP biomarker test as provided herein offers reliable detection of lactating cow pregnancies as early as at 17-19 days after insemination. The farmer may use this test as provided herein to improve reproduction management, reduce the number of cows that enter the less productive late lactation phase, and reduce the costs of unnecessary re-insemination. The source to detect the biomarker, milk, can be sampled without the costs of a veterinary service. Accurate pregnancy prediction at an early stage is economically important for dairy farmers. Currently the earliest pregnancy test available is at 35 days after insemination. Ovulation occurs at an interval of 21 days. Therefore, a pregnancy test before 21 days, e.g. 19 days after first insemination, is herein developed. Knowing the cow is not pregnant before the next heat will make farmers aware so they can keep an eye on the cow and act accordingly. This can reduce the calving interval of some cows with 21 days. Knowing the cow is pregnant will prevent unnecessary re-insemination. This will save insemination costs, but more importantly prevent damage to the zygote that could lead to early embryonic death.

If cows are not pregnant and the farmer doesn't know this he might miss the first possibility to re-inseminate and unnecessary increase the calving interval with 21 days. The economic analysis provided below considers a reduction in calving interval of 21 days for one cow.

Cost and revenue table

| Current average calving interval (d) | 416 |

(continued)

| Calving interval with early pregnancy test (d) | 395 |
|---|---|
| Average 305 days milk production (kg) | 8785 |
| Costs milk production losses (€/kg) | 0.12 |
| Average revenue of calf (€/cow) | 50 |
| Average cost dry-off (€/cow) | 20 |
| Average cost diseases due to calving (€/cow) | 60 |
| Average labour cost due to calving (€/cow) | 72 |
| Revenue reducing calving interval with 21 days (€/yr) | 10 |

Reducing the calving interval from the national average of 416 to 395 for non-pregnant cows increases the revenue by €10, - per cow per year. On an average Dutch farm with 90 cows and a non-return rate at 56 days of 66% there are 30.6 cows that might not be pregnant. Not missing their next heat and thus reducing their calving interval with 21 days will lead to a profit of € 306 per year.

In addition, farmer does not need to do their usual pregnancy testing. The test is much easier that rectal palpation or ultra-sound, as the cows do not need to be confined for a large part of the day and it is not labour intensive.

Tables

Table 1. Numbers of pregnant and non-pregnant cows per farm.

| Farm | Pregnant* cows | Non-pregnant* cows |
|---|---|---|
| 1 | 3 | 3 |
| 2 | 1 | 1 |
| 3 | 5 | 5 |
| 4 | 3 | 3 |
| 5 | 2 | 2 |
| 6 | 11 | 11 |
| 7 | 2 | 2 |
| 8 | 2 | 2 |
| 9 | 1 | 1 |
| * As defined above | | |

Table 2. Milk Progesterone measurements per pregnancy groups on day 19. P-value was determined with a T-test.

| Pregnancy Groups | Mean | SD | P-value |
|---|---|---|---|
| Pregnant (P) | 21.02 | 5.82 | |
| Not-Pregnant (NP) | 12.67 | 9.71 | |
| Not-Pregnant with Re-Insemination (NPRI) | 10.98 | 9.82 | |
| P-NP | | | 0.0078 |
| P-NPRI | | | 0.0022 |
| NP-NPRI | | | 0.65 |
| P-(NP+NPRI) | | | 0.00007 |

Table 3. Biomarker compositions composed of one to six components.

| No. of Components | Components | Mean Sensitivity | Mean Specificity | Mean Correctly classified |
|---|---|---|---|---|
| 1 | Progesterone | 0.80 | 0.67 | 0.73 |
| 2 | MFGM660; Progesterone | 0.82 | 0.80 | 0.81 |
| 3 | MS147; MS9; Progesterone | 0.86 | 0.85 | 0.85 |
| 4 | MS147; MS9; MFGM713; Progesterone | 0.92 | 0.93 | 0.92 |
| 5 | MS147; MS9; MFGM661; MFGM197; Progesterone | 0.96 | 0.93 | 0.94 |
| 6 | MS147; MS9; MFGM661; MFGM197; MS92; Progesterone | 0.96 | 0.96 | 0.96 |

Table 4. Biological information of the proteins included in the biomarkers.

| Protein fraction | Protein name | Embryo | Placenta | Mammary gland / lactation |
|---|---|---|---|---|
| MS147 | Kappa-casein (CSN3) | +/- | - | + |
| MFGM661 | Rho-related GTP-binding protein (RhoB) | +/- | + | + |
| MS9 | Protein disulfide-isomerase (P4HB) | + | + | - |
| MFGM197 | Arachidonate 12-lipoxygenase, (ALOX12) | + | - | - |
| MS92 | Cathepsin Z (CTSZ) | + | + | - |
| MFGM713 | Osteoclast-stimulating factor 1 (OSTF1) | + | + | - |
| +: Protein annotated with trait, -: protein not annotated with trait; +/- Protein weakly annotated with trait. | | | | |

Table 5 Results of 60 cows tested herein, with means and SD of measured values:

| Protein peak | Protein name | Mean not pregnant | SD not pregnant | N1 | Mean pregnant | SD pregnant | N2 |
|---|---|---|---|---|---|---|---|
| MFGM660 | UBXN4 | 6.077009 | 0.644978 | 30 | 6.317636 | 0.1764 49 | 28 |
| Melks147 | CSN3 | 8.042148 | 0.316377 | 28 | 7.719553 | 0.3730 38 | 29 |
| Melks9 | P4HB | 5.872109 | 0.110063 | 29 | 5.795756 | 0.0742 08 | 28 |
| MFGM713 | OSTF1 | 6.56852 | 0.70852 | 30 | 6.819031 | 0.1441 28 | 27 |
| MFGM661 | RhoB | 6.025698 | 0.632571 | 30 | 6.389319 | 0.2312 51 | 28 |
| MFGM197 | ALOX12 | 6.290528 | 0.839146 | 30 | 6.713969 | 0.2263 91 | 29 |
| Melks92 | CTSZ | 6.335653 | 0.175663 | 28 | 6.230273 | 0.2016 64 | 29 |
| Progesterone (ng/ml) | | 11.82333 | 9.96874 | 30 | 21.02367 | 5.9241 42 | 30 |

Table 6. Example of results

| Cow no. | CSN3 | P4HB | RhoB | ALOX12 | CTSZ | P4 | y= | Prediction |
|---|---|---|---|---|---|---|---|---|
| P1 | 7,45 | 5,81 | 6,57 | 6,84 | 6,29 | 34,64 | 465,44 | Pregnant |
| P2 | 7,37 | 5,83 | 6,40 | 6,72 | 6,40 | 32,41 | 400,01 | Pregnant |
| P3 | 7,26 | 5,66 | 6,37 | 6,73 | 6,22 | 19,43 | 386,30 | Pregnant |

(continued)

| Cow no. | CSN3 | P4HB | RhoB | ALOX12 | CTSZ | P4 | y= | Prediction |
|---|---|---|---|---|---|---|---|---|
| P4 | 7,52 | 5,75 | 6,38 | 7,07 | 6,22 | 26,19 | 377,34 | Pregnant |
| P5 | 7,34 | 5,70 | 6,37 | 6,95 | 6,28 | 17,53 | 327,23 | Pregnant |
| P6 | 8,44 | 5,83 | 6,57 | 6,99 | 6,26 | 23,20 | 35,97 | Pregnant |
| P7 | 8,24 | 5,80 | 6,20 | 6,90 | 6,14 | 20,48 | 32,99 | Pregnant |
| P8 | 8,59 | 5,81 | 6,61 | 6,71 | 6,27 | 25,09 | 21,12 | Pregnant |
| P9 | 7,95 | 5,68 | 5,93 | 6,27 | 6,09 | 13,56 | 21,06 | Pregnant |
| P10 | 7,49 | 5,90 | 6,33 | 6,47 | 6,61 | 16,66 | 20,45 | Pregnant |
| NP1 | 7,62 | 5,99 | 4,50 | 4,50 | 6,34 | 9,96 | -577,85 | Not Pregnant |
| NP2 | 7,90 | 6,01 | 6,11 | 6,17 | 6,38 | 0,05 | -494,25 | Not Pregnant |
| NP3 | 8,34 | 5,79 | 4,50 | 6,85 | 6,30 | 0,31 | -476,84 | Not Pregnant |
| NP4 | 8,26 | 5,98 | 6,30 | 6,74 | 6,14 | 0,05 | -475,86 | Not Pregnant |
| NP5 | 8,13 | 5,75 | 6,16 | 4,50 | 6,10 | 0,05 | -457,31 | Not Pregnant |
| NP6 | 8,45 | 5,69 | 6,23 | 6,77 | 6,33 | 14,45 | -26,35 | Not Pregnant |
| NP7 | 7,62 | 5,65 | 6,52 | 4,50 | 6,30 | 11,16 | -23,00 | Not Pregnant |
| NP8 | 8,04 | 6,06 | 6,52 | 6,78 | 6,56 | 29,41 | -21,64 | Not Pregnant |
| NP9 | 7,82 | 5,63 | 5,89 | 4,50 | 6,43 | 17,64 | -21,61 | Not Pregnant |
| NP10 | 7,94 | 5,84 | 6,27 | 6,83 | 6,37 | 15,20 | -19,90 | Not Pregnant |

Table 7 Commercially available ELISA

| kits. | | |
|---|---|---|
| Antibodies directed against | mybiosource.com | antikorper-online.de |
| CSN3 | MBS7200256 | ABIN1118134 |
| RhoB | MBS755041 | ABIN995301 |
| P4HB | MBS737735 | ABIN848162 |
| ALOX12 | MBS069425 | ABIN1122336 |
| CTSZ | MBS107875 | ABIN1771637 |
| OSTF1 | MBS034850 | ABIN004309 |

References

Ambrose, D. J., B. Radke, P. A. Pitney, and L. A. Goonewardene. 2007. Evaluation of early conception factor lateral flow test to determine nonpregnancy in dairy cattle. Can. Vet. J. 48:831-835.

Balhara, A. K., M. Gupta, S. Singh, A. K. Mohanty,.and I. Singh. 2013. Early Pregnancy Diagnosis in Bovines: Current Status and Future Directions. The Scientific World Journal 2013: Article ID 958540, 10 pages. http://dx.doi.org/10.1155/2013/958540.

Bulman, D. C. and G. E. Lamming. 1978. Milk progesterone levels in relation to conception, repeat breeding and factors influencing acyclicity in dairy cows. J. Reprod. Fertil. 54:447-458. Cordoba, M. C., R. Sartori, P. M. Fricke. 2001. Assessment of a commercially available early conception factor (ECF) test for determining pregnancy status of dairy cattle. J. Dairy Sci. 84:1884-1889.

Cox J., M. Mann. 2008. MaxQuant enables high peptide identification rates, individualized p.p.b.-range mass accuracies and proteome-wide protein quantification. Nature Biotechnol. 26s:1367-1372.

Cox J., N. Neuhauser, A. Michalski, R. A. Scheltema, J. V. Olsen, M. Mann. 2011. Andromeda: A Peptide Search Engine Integrated into the MaxQuant Environment. J Proteome Res. 10:1794-1805.

Gillis, E. H., I. Traynor, J. P. Gosling, M. Kane. 2006. Improvements to a surface plasmon resonance-based immunoassay for the steroid hormone progesterone. J. AOAC Int. 89:838-842.

Green, J. A., S. Xie, X. Quan, B. Bao, X. Gan, N. Mathialagan, J-F. Beckers, and R. M. Roberts. 2000. Pregnancy-Associated Bovine and Ovine Glycoproteins Exhibit Spatially and Temporally Distinct Expression Patterns During Pregnancy. Biol. Reprod. 62:1624-1631. Lee, A., B. Robertson, and M. A. Lee. 2012. Package 'R330'.

Lu, J., S. Boeren, S. C. de Vries, H. J. F. van Valenberg, J. Vervoort, and K. Hettinga. 2011. Filter-aided sample preparation with dimethyl labeling to identify and quantify milk fat globule membrane proteins. J. Prot 74:34-43.

Lucy, M. C. 2001. ADSA Foundation Scholar Award - Reproductive loss in high-producing dairy cattle: Where will it end? J. Dairy Sci. 84:1277-1293.

Memili, E. and N. L. First. 1999. Zygotic and embryonic gene expression in cow: a review of timing and mechanisms of early gene expression as compared with other species. Zygote 8:87-96.

Osman, M. M., K. H. M. El Bayomi, A. S. Abdoon, and A. A. S. El Nabtiti. 2012. Determination of the cutoff point of milk progesterone and use it as an early indicator of pregnancy or screening of open cows in dairy farms. Vet. Sci. Egypt. 17:19-31.

Petit, H. V. 2003. Digestion, Milk Production, Milk Composition, and Blood Composition of Dairy Cows Fed Formaldehyde Treated Flaxseed or Sunflower Seed. J. Dairy Sci. 86:2637-2646.

R Core Team (2014). R: A language and environment for statistical computing. R Foundation for Statistical Computing, Vienna, Austria. http://www.R-project.org/.

Silke, V., M. G. Diskin, D. A. Kenny, M. P. Boland, P. Dillon, J. F. Mee, J. M. Sreenan. 2002. Extent, pattern and factors associated with late embryonic loss in dairy cows. Anim. Reprod. Sci. 71:1-12.

Smaczniak, C., N. Li, S. Boeren, T. America, W. van Dongen, S. S. Goerdayal, S. de Vries, G. C. Angenent, K. Kaufmann. 2012. Proteomics-based identification of low-abundance signaling and regulatory protein complexes in native plant tissues. Nature Protocols 7:2144-2158.

Stelzl, U., U. Worm, M. Lalowski, C. Haenig, F. H. Brembeck, H. Goehler, M. Stroedicke, M. Zenkner, A. Schoenherr, S. Koeppen, J. Timm, S. Mintzlaff, C. Abraham, N. Bock, S. Kietzmann, A. Goedde, E. Toksöz, A. Droege, S. Krobitsch, B. Korn, W. Birchmeier, H. Lehrach, and E. E. Wanker. 2005. A Human Protein-Protein Interaction Network: A Resource for Annotating the Proteome. Cell 122:957-968.

Szenci, O., J. F. Beckers, P. Humblot, J. Sulon, G. Sasser, M. A. M. Taverne, J. Varga, R. Baltusen, and G. Schekk. 1998. Comparison of ultrasonography, bovine pregnancy-specific protein B, and bovine pregnancy-associated glycoprotein 1 tests for pregnancy detection in dairy cows. Theriogenology, 50:77-88.

Te Pas, M. F. W., L. Kruijt, S-J. Koopmans, M. A. Smits. 2013a. Plasma Proteome profiles associated with diet-induced metabolic syndrome and the early onset of metabolic syndrome in a pig model. PLoS ONE 8 Issue 9, e73087.

Te Pas, M. F. W., L. Kruijt, M. Pierzchala, R. E. Crump, S. Boeren E. Keuning, R. A. Hoving-Bolink, M. Hortós, M. Gispert J. Arnau, A. Diestre, H. A. Mulder. 2013b.Identification of proteomic biomarkers in M. Longissimus dorsi as potential predictors of pork quality. Meat Sci. 95:679-687.

Vasconcelos, J. L. M., R. W. Silcox, J. A. Lacerda, J. R. Pursley, M. C. Wiltbank. 1997. Pregnancy rate, pregnancy loss, and response to head stress after AI at 2 different times from ovulation in dairy cows. Biol. Reprod. 56:230-230.

Yoshida, C. and T. Nakao. 2005. Some characteristics of primary and secondary oestrous signs in high-producing dairy cows. Reprod. Domest. Anim. 40:150-155.

## Claims

1. A method to determine whether an inseminated female mammal is pregnant prior to a point in time at which said mammal would be expected to ovulate if non-pregnant, said method comprising taking a biological sample from said mammal prior to said point in time and testing said sample for the presence of a multiple pregnancy marker pattern (MPMP).

2. A method according to claim 1 wherein said sample is tested for the relative expression of progesterone and of at least one marker selected from a group of marker proteins that each contribute to a significantly differential marker expression pattern related to the status pregnancy.

3. A method according to claim 1 or 2 wherein said sample is tested to determine whether said mammal is non-pregnant and may be re-inseminated.

4. A method according to anyone of claims 1 to 3 wherein a marker selected from said group is identified with a false discovery rate of less than 1%.

5. A method according to anyone of claims 1 to 4 wherein said expression of said at least one marker is generally detectable in biological samples of female mammals of the species tested.

6. A method according to anyone of claims 1 to 5 wherein testing the relative expression of at least one marker selected from said group of biomarker proteins increases the predictive value of determining pregnancy by testing the relative expression of progesterone of said sample.

7. A method according to anyone of claims 1 to 6 wherein testing said sample is done by mass spectrometry.

8. A method according to claim 7 wherein testing said sample is done by LC-MS/MS spectrometry.

9. A method according to anyone of claims 1 to 6 wherein testing said sample is done by immunoassay.

10. A method according to claim 9 wherein testing said sample is done by a multiplex immunoassay.

11. A method according to anyone of claims 1 to 10 and said sample is milk.

12. A method according to anyone of claims 1 to 11 wherein said mammal is tested prior to a point in time at which said mammal would be expected to present with standing heat if non-pregnant.

13. A method according to anyone of claims 1 to 11 wherein said mammal is a human and inseminated at least at about 28 days prior to said point in time.

14. A method according to anyone of claims 1 to 12 wherein said mammal is a goat and inseminated at least at about 19 days prior to said point in time.

15. A method according to anyone of claims 1 to 12 wherein said mammal is a sheep and inseminated at least at about 19 days prior to said point in time.

16. A method according to anyone of claims 1 to 12 wherein said mammal is a pig and inseminated at least at about 21 days prior to said point in time.

17. A method according to anyone of claims 1 to 12 wherein said mammal is a horse and inseminated at least at about 21 days prior to said point in time.

18. A method according to anyone of claims 1 to 12 wherein said mammal is a cow and inseminated at least at about 21 days prior to said point in time.

19. A method according to claim 18 wherein said sample is taken from 17 - 21 days, preferably from 18 - 20 days, more preferably at 19 days after insemination of said cow.

20. A method according to claim 18 or 19 wherein said group of marker proteins comprises kappa-casein (CSN3), Rho-related GTP-binding protein (RhoB), Protein disulfide-isomerase (P4HB), Arachidonate 12-lipoxygenase (ALOX12), Cathepsin Z (CTSZ), Osteoclast-stimulating factor 1 (OSTF1) and UBX domain protein 4 (UBXN4).

21. A method according to claim 20 wherein said of at least one marker comprises UBXN4.

22. A method according to claim 20 wherein said of at least one marker comprises CSN3 and P4HB.

23. A method according to claim 20 wherein said of at least one marker comprises CSN3, P4HB and OSTF1.

24. A method according to claim 20 wherein said of at least one marker comprises CSN3, P4HB, RhoB, and ALOX12.

25. A method according to claim 20 wherein said of at least one marker comprises CSN3, P4HB, RhoB, ALOX12 and CTSZ.

26. A kit of parts comprising detection means of one or more markers selected from the group consisting of kappa-casein (CSN3), Rho-related GTP-binding protein (RhoB), Protein disulfide-isomerase (P4HB), Arachidonate 12-lipoxygenase, (ALOX12), Cathepsin Z (CTSZ), Osteoclast-stimulating factor 1 (OSTF1) and UBX domain protein 4 (UBXN4).

27. A kit of parts according to claim 26, further comprising detection means of progesterone.

28. Use of a kit of parts according to claim 26 or 27 for determining whether a cow is pregnant.

29. Use of detection means of one or more markers selected from the group consisting of kappa-casein (CSN3), Rho-related GTP-binding protein (RhoB), Protein disulfide-isomerase (P4HB), Arachidonate 12-lipoxygenase, (ALOX12), Cathepsin Z (CTSZ), Osteoclast-stimulating factor 1 (OSTF1) and UBX domain protein 4 (UBXN4), for determining the presence and/or (relative) amount of one or more of said markers in a biological sample, preferably in cows milk.

Figure 1

OVULATION

|←————— Standing Heat 15–18 Hours —————→|      ↓   Fertile Life of Egg 10–20 Hours

|←—— Optimum Time to Inseminate →|     Most Fertile Period
8–10 Hours

|←———— Sperm Life in Female Tract 18–24 Hours ————→|

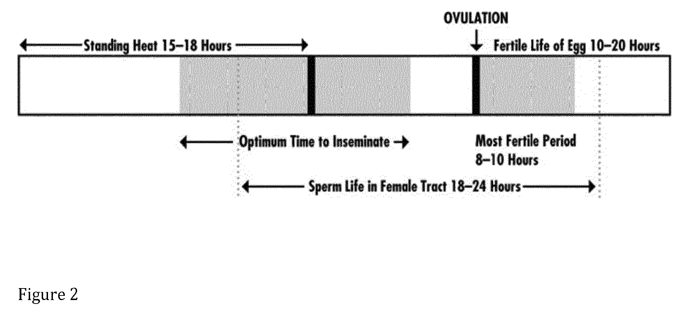

Figure 2

Europäisches Patentamt

European Patent Office

Office européen des brevets

## EUROPEAN SEARCH REPORT

Application Number

EP 16 16 1039

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ASHOK K. BALHARA ET AL: "Early Pregnancy Diagnosis in Bovines: Current Status and Future Directions", THE SCIENTIFIC WORLD JOURNAL, vol. 78, no. 1-2, 1 January 2013 (2013-01-01), pages 143-10, XP055328062, GB, NL ISSN: 1537-744X, DOI: 10.1016/S0009-8981(02)00186-9 * whole document, in particular table 2, paragraph 2.2 (pages 2-5) and paragraph 3 (pages 5 and 6) . * | 1-15 | INV. G01N33/68 G01N33/74 |
| X | JAE EUN LEE ET AL: "Proteomic Analysis of Bovine Pregnancy-specific Serum Proteins by 2D Fluorescence Difference Gel Electrophoresis", ASIAN-AUSTRALASIAN JOURNAL OF ANIMAL SCIENCES., vol. 28, no. 6, 26 March 2015 (2015-03-26) , pages 788-795, XP055328381, KR ISSN: 1011-2367, DOI: 10.5713/ajas.14.0790 * Whole document, in particular abstract and Table 2 * | 1,3,5,7, 12 | |
| X | WO 2004/059282 A2 (MONSANTO TECHNOLOGY LLC [US]; NAGAPPAN MATHIALAGAN [US]) 15 July 2004 (2004-07-15) * Whole document, in particular claims and the examples * | 1-15 | TECHNICAL FIELDS SEARCHED (IPC) |
| X | WO 03/043524 A2 (UNIV MISSOURI [US]; MONSANTO TECHNOLOGY LLC [US]; LUCY MATTHEW C [US];) 30 May 2003 (2003-05-30) * whole document, in particular claims and examples * | 1-15 | G01N |

The present search report has been drawn up for all claims

| Place of search | Date of completion of the search | Examiner |
|---|---|---|
| Munich | 13 December 2016 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 16 16 1039

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☒ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

## ANNEX TO THE EUROPEAN SEARCH REPORT
## ON EUROPEAN PATENT APPLICATION NO.

EP 16 16 1039

13-12-2016

| Patent document cited in search report | | | Publication date | Patent family member(s) | | | Publication date |
|---|---|---|---|---|---|---|---|
| WO 2004059282 | A2 | | 15-07-2004 | AU | 2003297304 | A1 | 22-07-2004 |
| | | | | WO | 2004059282 | A2 | 15-07-2004 |
| WO 03043524 | A2 | | 30-05-2003 | AU | 2002366097 | A1 | 10-06-2003 |
| | | | | CA | 2467904 | A1 | 30-05-2003 |
| | | | | EP | 1468288 | A2 | 20-10-2004 |
| | | | | MX | PA04004880 | A | 03-09-2004 |
| | | | | US | 2006199235 | A1 | 07-09-2006 |
| | | | | WO | 03043524 | A2 | 30-05-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AMBROSE, D. J. ; B. RADKE ; P. A. PITNEY ; L. A. GOONEWARDENE.** Evaluation of early conception factor lateral flow test to determine nonpregnancy in dairy cattle. *Can. Vet. J.,* 2007, vol. 48, 831-835
- **BALHARA, A. K. ; M. GUPTA ; S. SINGH ; A. K. MOHANTY ; I. SINGH.** Early Pregnancy Diagnosis in Bovines: Current Status and Future Directions. *The Scientific World Journal,* 2013, 10, http://dx.doi.org/10.1155/2013/958540
- **BULMAN, D. C. ; G. E. LAMMING.** Milk progesterone levels in relation to conception, repeat breeding and factors influencing acyclicity in dairy cows. *J. Reprod. Fertil.,* 1978, vol. 54, 447-458
- **CORDOBA, M. C. ; R. SARTORI ; P. M. FRICKE.** Assessment of a commercially available early conception factor (ECF) test for determining pregnancy status of dairy cattle. *J. Dairy Sci.,* 2001, vol. 84, 1884-1889
- **COX J. ; M. MANN.** MaxQuant enables high peptide identification rates, individualized p.p.b.-range mass accuracies and proteome-wide protein quantification. *Nature Biotechnol.,* 2008, vol. 26s, 1367-1372
- **COX J. ; N. NEUHAUSER ; A. MICHALSKI ; R. A. SCHELTEMA ; J. V. OLSEN ; M. MANN.** Andromeda: A Peptide Search Engine Integrated into the MaxQuant Environment. *J Proteome Res.,* 2011, vol. 10, 1794-1805
- **GILLIS, E. H. ; I. TRAYNOR ; J. P. GOSLING ; M. KANE.** Improvements to a surface plasmon resonance-based immunoassay for the steroid hormone progesterone. *J. AOAC Int.,* 2006, vol. 89, 838-842
- **GREEN, J. A. ; S. XIE ; X. QUAN ; B. BAO ; X. GAN ; N. MATHIALAGAN ; J-F. BECKERS ; R. M. ROBERTS.** Pregnancy-Associated Bovine and Ovine Glycoproteins Exhibit Spatially and Temporally Distinct Expression Patterns During Pregnancy. *Biol. Reprod.,* 2000, vol. 62, 1624-1631
- **LEE, A. ; B. ROBERTSON ; M. A. LEE.** *Package 'R330,* 2012
- **LU, J. ; S. BOEREN ; S. C. DE VRIES ; H. J. F. VAN VALENBERG ; J. VERVOORT ; K. HETTINGA.** Filter-aided sample preparation with dimethyl labeling to identify and quantify milk fat globule membrane proteins. *J. Prot,* 2011, vol. 74, 34-43

- **LUCY, M. C.** ADSA Foundation Scholar Award - Reproductive loss in high-producing dairy cattle: Where will it end?. *J. Dairy Sci.,* 2001, vol. 84, 1277-1293
- **MEMILI, E. ; N. L. FIRST.** Zygotic and embryonic gene expression in cow: a review of timing and mechanisms of early gene expression as compared with other species. *Zygote,* 1999, vol. 8, 87-96
- **OSMAN, M. M. ; K. H. M. EL BAYOMI ; A. S. ABDOON ; A. A. S. EL NABTITI.** Determination of the cutoff point of milk progesterone and use it as an early indicator of pregnancy or screening of open cows in dairy farms. *Vet. Sci. Egypt.,* 2012, vol. 17, 19-31
- **PETIT, H. V.** Digestion, Milk Production, Milk Composition, and Blood Composition of Dairy Cows Fed Formaldehyde Treated Flaxseed or Sunflower Seed. *J. Dairy Sci.,* 2003, vol. 86, 2637-2646
- R: A language and environment for statistical computing. *R Foundation for Statistical Computing, Vienna, Austria,* 2014, http://www.R-project.org
- **SILKE, V. ; M. G. DISKIN ; D. A. KENNY ; M. P. BOLAND ; P. DILLON ; J. F. MEE ; J. M. SREENAN.** Extent, pattern and factors associated with late embryonic loss in dairy cows. *Anim. Reprod. Sci.,* 2002, vol. 71, 1-12
- **SMACZNIAK, C. ; N. LI ; S. BOEREN ; T. AMERICA ; W. VAN DONGEN ; S. S. GOERDAYAL ; S. DE VRIES ; G. C. ANGENENT ; K. KAUFMANN.** Proteomics-based identification of low-abundance signaling and regulatory protein complexes in native plant tissues. *Nature Protocols,* 2012, vol. 7, 2144-2158
- **STELZL, U. ; U. WORM ; M. LALOWSKI ; C. HAENIG ; F. H. BREMBECK ; H. GOEHLER ; M. STROEDICKE ; M. ZENKNER ; A. SCHOENHERR ; S. KOEPPEN.** A Human Protein-Protein Interaction Network: A Resource for Annotating the Proteome. *Cell,* 2005, vol. 122, 957-968
- **SZENCI, O. ; J. F. BECKERS ; P. HUMBLOT ; J. SULON ; G. SASSER ; M. A. M. TAVERNE ; J. VARGA ; R. BALTUSEN ; G. SCHEKK.** Comparison of ultrasonography, bovine pregnancy-specific protein B, and bovine pregnancy-associated glycoprotein 1 tests for pregnancy detection in dairy cows. *Theriogenology,* 1998, vol. 50, 77-88

- **TE PAS ; M. F. W. ; L. KRUIJT ; S-J. KOOPMANS ; M. A. SMITS.** Plasma Proteome profiles associated with diet-induced metabolic syndrome and the early onset of metabolic syndrome in a pig model. *PLoS ONE,* 2013, vol. 8 (9), e73087
- **TE PAS ; M. F. W. ; L. KRUIJT ; M. PIERZCHALA ; R. E. CRUMP ; S. BOEREN ; E. KEUNING ; R. A. HOVING-BOLINK ; M. HORTÓS ; M. GISPERT.** Identification of proteomic biomarkers in M. Longissimus dorsi as potential predictors of pork quality. *Meat Sci.,* 2013, vol. 95, 679-687
- **VASCONCELOS, J. L. M. ; R. W. SILCOX ; J. A. LACERDA ; J. R. PURSLEY ; M. C. WILTBANK.** Pregnancy rate, pregnancy loss, and response to head stress after AI at 2 different times from ovulation in dairy cows. *Biol. Reprod.,* 1997, vol. 56, 230-230
- **YOSHIDA, C. ; T. NAKAO.** Some characteristics of primary and secondary oestrous signs in high-producing dairy cows. *Reprod. Domest. Anim.,* 2005, vol. 40, 150-155